Europäisches Patentamt

European Patent Office

Office européen des brevets

(11) Publication number: **0 004 719**
**A2**

## (12) EUROPEAN PATENT APPLICATION

(21) Application number: **79300425.0**

(22) Date of filing: **19.03.79**

(51) Int. Cl.²: **A 61 K 7/22**
**A 61 K 7/24**

(30) Priority: **31.03.78 US 892276**

(43) Date of publication of application:
**17.10.79 Bulletin 79/21**

(84) Designated Contracting States:
**BE DE FR GB IT NL SE**

(71) Applicant: **BEECHAM INC.**
**65 Industrial South**
**Clifton New Jersey 07012(US)**

(72) Inventor: **Rodon, Maria**
**3113 Sherry Drive**
**Raleigh North Carolina 27604(US)**

(74) Representative: **Walls, Alan James et al,**
**Beecham Pharmaceuticals Yew Tree Bottom Road**
**Epsom, Surrey, KT18 5XQ(GB)**

(54) Oral hygiene compositions.

(57) Oral hygiene compositions useful for controlling dental plaque and gingivitis and for preventing or reducing caries are produced comprising a cationic antimicrobial agent, such as alexidine dihydrochloride, and malic acid as anti-stain agent which reduces the tooth staining effect of the cationic antimicrobial.

EP 0 004 719 A2

0004719

.TITLE MODIFIED
see front page

TITLE: Oral Compositions

The present invention relates to oral hygiene compositions useful for controlling dental plaque and gingivitis and for preventing or reducing caries.

It is known that certain cationic antimicrobial agents are capable of controlling dental plaque and gingivitis and for preventing or reducing caries when applied regularly to the teeth and oral cavity. Such active agents include certain bisbiguanides and quaternary ammonium compounds. However, these active ingredients have the disadvantage that they stain or discolour the surfaces of teeth.

For example it is known in the art that bisbiguanides inhibit the formation of plaque and caries (see U.S. Patent No. 1,365,030) and that certain anti-calculus agents may be combined therewith to inhibit the tendency of the bisbiguanides to stain the oral surfaces (see U.S. Patent No. 3,934,002). Among the anti-calculus agents disclosed in U.S. Patent No. 3,934,002 are quaternary ammonium compounds, zinc phenolsulfonate, hydroxyquinoline, citric acid, lactic acid and pharmaceutically acceptable salts thereof.

The present invention is based on the surprising discovery that malic acid markedly inhibits the staining of tooth surfaces by such bisbiguanides or quaternary ammonium compounds having similar activities.

Accordingly, the present invention provides an oral hygiene composition, useful for controlling dental plaque and gingivitis and for preventing or reducing caries which comprises a bisbiguanide or quaternary ammonium compound, which is capable of controlling dental plaque and gingivitis and preventing or reducing caries, and malic acid.

The amount of bisbiguanide or quaternary ammonium compound may be from 0.01% to about 1.0% w/w based on the total weight of ingredients of the composition or w/v when in liquid form. When the composition is in the form of a concentrate for dilution before application to the oral acvity, larger amounts of the active ingredient may be present.

The amount of malic acid may be from 0.1% to 10.0% w/w or w/v, especially from 0.1% to 1.0%. Likewise, if the composition is to be diluted before use, larger amounts of malic acid may be present.

The pH of the composition may be from about 5.0 to about 8.0.

The bisbiguanide may be 1,6-bis (2-ethylhexyldiguanido hexane) dihydrochloride [alexidine dihydrochloride]; 1,6-bis (2-ethylhexyl diguanido hexane) dihydrofluoride; 1,6-bis (2-ethylhexyl diguanido octane) dihydrochloride; 1,6-bis (2-ethylhexyl diguanido nonane) dihydrochloride; 1,6-bis (2-ethylhexyl diguanido dodecane) dihydrochloride; or 1,6-di (4-chlorophenyl diguanido hexane) dihydrochloride or the diacetate or digluconate salt thereof. Alexidine dihydrochloride is especially preferred.

The quaternary ammonium compound may be dodecyl dimethyl-(2-phenoxyethyl)-ammonium; benzyldimethyl (2-)2-(p-1,3,3-tetramethyl butyl phenoxy)ethyl)ammonium; p-bromo-benzyldimethyl-γ-(2'-isopropyl, 4'-chloro, 5'-methyl phenoxy)-propyl ammonium; 1-hexadecyl-pyridinium salt; acylchloamino-formylmethyl pyridinium chloride-iodide complex; 1-alkyl-4-aminoquinaldinium salt; decamethylene bis(4-aminoquinaldinum chloride) or hexadecamethylene bis (isoquinolinium chloride).

The usual flavouring agents, binders, sudsing agents, humectants, alcohols, fragrances, abrasives and excipients known in the art can be added to the compositions of the present invention.

When the composition of the present invention is in the form of a mouthwash, oral rinse or gargle, the composition is brought into contact with the oral cavity and then expectorated. A dose of 15 to 20 ml. for adults and about 10 ml. for children is generally sufficient when used on a daily basis, and when the concentration of active bisbiguanide or quaternary ammonium compound is in the range 0.01 to 1.0% w/w or w/v.

When the instant composition is in the form of a dentifrice, such as a paste, powder, concentrate, solution or gel for direct application to the teeth, it can be used in the normal manner in which a toothpaste is used. When the oral composition of the present invention is in a concentrate for use with mechanical irrigation devices such as a water jet or "water pik" type device, approximately 10 to 15 ml. should be sprayed into the mouth and circulated in the oral cavity and then be expectorated. When the present composition is in the form of a breath freshener, either pump spray or aerosol type, approximately 10 to 15 ml. should be sprayed into the mouth, circulated therethrough and expectorated. When the composition of the present invention is in the form of a troche or a lozenge, it should be allowed to dissolve in the mouth and then be expectorated.

The following nonlimitative example more particularly illustrates the present invention.

| Ingredients | Per cent w/v |
|---|---|
| Alexidine Dihydrochloride | 0.035 |
| Alcohol USP | 15.0 |
| Glycerin USP | 10.0 |
| Flavour | 0.4 |
| Sodium Saccharin | 0.02 |
| Malic Acid | 0.382 |
| Buffering Agent to pH 5.0-8.0 | |
| Water USP | q.s. |

The mouth-rinse is used in 15 ml. doses for adults to rinse the mouth and then is expectorated. For children in the 6 to 12 year old range, 10ml. is the recommended mouth-rinsing dose.

CLAIMS

1.    An oral hygiene composition, useful for controlling dental plaque and gingivitis and for preventing or reducing caries which comprise a bisbiguanide or quaternary ammonium compound, which is capable of controlling dental plaque and gingivitis and preventing or reducing caries, and malic acid.

2.    A composition according to claim 1 wherein the amount of bisbiguanide or quaternary ammonium compound is from 0.01% to about 1.0% w/w or w/v, based on the total weight of ingredients of the composition.

3.    A composition according to claim 1 or claim 2 wherein the amount of malic acid is from 0.1% to 10.0% w/w or w/v.

4.    A composition according to claim 3 wherein the amount of malic acid is from 0.1% to 1.0% w/w or w/v.

5.    A composition according to any one of the preceding claims wherein the pH of the composition is from about 5.0 to about 8.0.

6.    A composition according to any one of the preceding claims wherein a bisbiguanide is present and is 1,6-bis (2-ethylhexyl diguanido hexane) dihydrochloride; 1,6-bis (2-ethylhexyl diguanido hexane) dihydrofluoride; 1,6-bis (2-ethylhexyl diguanido octane) dihydrochloride; 1,6-bis (2-ethylhexyl diguanido nonane) dihydrochloride; or 1,6-di (4-chlorophenyl diguanido hexane) dihydrochloride or the diacetate or digluconate salt thereof.

7.    A composition according to any one of claims 1 to 5 wherein a quaternary ammonium compound is present and is dodecyl dimethyl-(2-phenoxy-ethyl)-ammonium; benzyldimethyl (2-)2-(p-1,3,3-tetramethyl butyl phenoxy)ethoxyl)ethyl) ammonium; p-bromobenzyl-dimethyl-γ-(2'-isopropyl, 4'-chloro, 5'-methyl phenoxy)-propyl ammonium; 1-hexadecyl-pyridinium salt; acylcholaminoformyl-methyl pyridinium chloride-iodide complex; 1-alkyl-4-aminoquinaldinium salt; decamethylene bis(4-aminoquinaldinum chloride) or hexadecamethylene bis (isoquinolinium chloride).

0004719

8.      A composition according to claim 6 wherein the antimicrobial agent is 1,6-bis (2-ethylhexyl diguanido hexane) dihydrochloride.

9.      A composition according to any one of the preceding claims in the form of a mouthwash, oral rinse, dentifrice, toothpowder, aerosol, troche or lozenge, gel, or in a form suitable for dispensing through a water jet.

10.      A composition as claimed in claim 1, substantially as hereinbefore described in the Example.